# EUROPEAN PATENT SPECIFICATION

(11) **EP 0 968 987 B1**
(45) Date of publication and mention of the grant of the patent: **04.02.2004**
(21) Application number: 99110816.8
(22) Date of filing: 05.06.1999
(51) Int. Cl.: C07C 13/40, C10M 105/04

(54) **Derivatives of bicyclo[2.2.1]heptane, method for their production, and traction fluid**
Derivate von Bicyclo[2.2.1]heptan, Verfahren zu ihrer Herstellung und deren Verwendung als Antreibflüssigkeit
Dérivés de bicyclo[2.2.1]heptane, procédé pour leur préparation et leur utilisation comme fluide de traction

(30) Priority: 01.07.1998 JP 18639998
(43) Date of publication of application: 05.01.2000
(73) Proprietor: Idemitsu Kosan Co., Ltd., Tokyo 100-8321 (JP)
(72) Inventor: Yoshida, Yukio, c/o Idemitsu Kosan Co.,Ltd., Sodegaura-shi, Chiba 299-0293 (JP); Tsubouchi, Toshiyuki, c/o Idemitsu Kosan Co.,Ltd., Sodegaura-shi, Chiba 299-0293 (JP); Ido, Motohisa, c/o Idemitsu Kosan Co.,Ltd., Sodegaura-shi, Chiba 299-0293 (JP); Hata, Hitoshi, Idemitsu Kosan Co., Ltd., Ichihara-shi, Chiba 299-0107 (JP)
(74) Representative: Gille Hrabal Struck Neidlein Prop Roos

(56) References cited:
- EP-A- 0 402 881

## Description

### Technical Field of the Invention

The present invention relates to derivatives of bicyclo [2. 2. 1] heptane with a special structure, and particularly to derivatives of bicyclo [2. 2. 1] heptane with a special structure having a high traction coefficient under high temperature and an excellent viscosity characteristic under low temperature and therefore useful for fluid for a traction drive, and a method for production of such useful derivatives of bicyclo [2. 2. 1] heptane with a special structure economically and efficiently.

In addition, the invention relates to fluid for a traction drive, and particularly fluid for a traction drive having a high traction coefficient under high temperature and an excellent viscosity characteristic under low temperature.

### Related Art

Fluid for a traction drive is used in such machine for traction drive as a continuously variable transmission for automobiles and a continuously variable transmission for industry as a lubricating oil to drive them.

A traction coefficient required for the fluid for traction drive is generally known as depending on the size of the traction drive. The fluid for traction drive with a higher traction coefficient is required for developing a small and light traction drive machine.

For example, in CVT (continuously variable transmission) of traction type that is developed as a continuously variable transmission for automobiles, the fluid for traction drive used for CVT requires to have enough high traction coefficient under high temperature to reach, for example, the lowest within a range of applied temperature than a designed temperature for CVT in order to make size of the CVT small and increase the capacity of its torque transmission.

Besides, the fluid for traction drive requires various performances other than the higher traction coefficient.

A low viscosity, for example, 150 Pa·s or lower is required, for example, at -40 °C to keep excellent starting performance in a cold region.

On the other hand, it is required to suppress volatilization of base oil and keep enough thickness of an oil layer for use under high temperature.

Among them, increasing the traction coefficient under the high temperature does not support decreasing the viscosity under the low temperature. Developing traction oil to realize both these performances was very difficult. Against such background, the inventors of the invention enthusiastically studied and discovered a group of compounds excellent in the traction coefficient under the high temperature (JP 03 095295 A corresponding to EP-A-0 402 881). However, those compounds showed insufficient characteristics of viscosity under low temperature.

### SUMMARY OF THE INVENTION

The invention was achieved in the view aforementioned and for the purpose of providing derivatives of bicyclo [2. 2. 1] heptane with a high traction coefficient under high temperature and an excellent viscosity characteristic under low temperature and useful for the fluid for a traction drive and also providing a method for producing such derivatives economically and efficiently.

Besides, the invention was achieved for the purpose of providing the fluid for traction drive, having a high traction coefficient under high temperature and an excellent viscosity characteristic under low temperature.

As a result of a research series of the inventors, who discovered that derivatives of bicyclo [2.2.1] heptane with a special structure show particularly excellent performances as the fluid for traction drive and that the derivatives of bicyclo [2.2.1] heptane can be economically and efficiently prepared by dimerization of a special olefinic alicyclic ring compound as a material under a special reaction condition and then by hydrogenation.

In addition, the inventors found as a result of a series of research that a synthetic oil with a special property shows an excellent performance as the fluid for traction drive.

The invention has been completed based on these findings.

The followings are the abstract of the invention.
(1) Exo-2-methyl-exo-3-methyl-endo-2-[(endo-2-methylbicyclo [2.2.1]hept-exo-3-yl)methyl]bicyclo[2.2.1]heptane of formula (I), 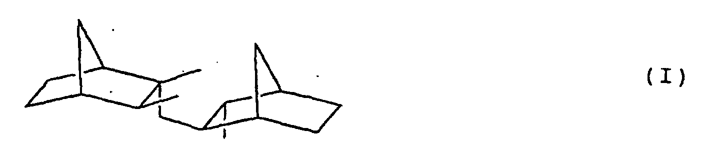 Exo-2-methyl-exo-3-methyl-endo-2[(endo-3-methylbicyclo[2.2.1]-hept-exo-2-yl)methyl] bicyclo[2.2.1]heptane of formula (II), 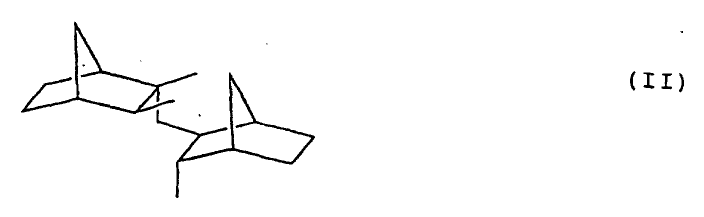 endo-2-methyl-exo-3-methyl-exo-2-[(exo-3-methylbicyclo[2.2.1]hept-exo-2-yl)methyl]bicyclo[2.2.1]heptane of formula (III), 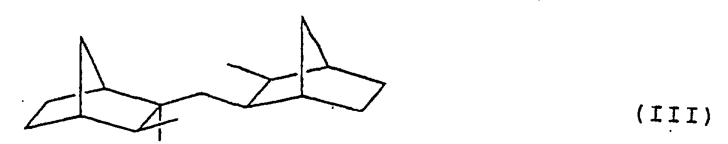 endo-2-methyl-exo-3-methyl-exo-2-[(exo-2-methylbicyclo[2.2.1]-hept-exo-3-yl)methyl]bicyclo[2.2.1]heptane of formula (IV), 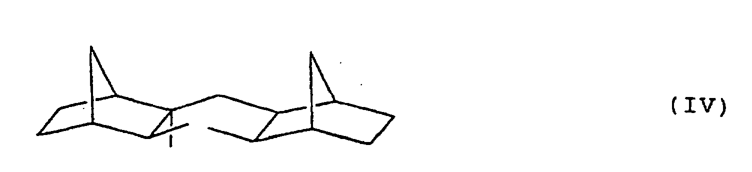 endo-2-methyl-exo-3-methyl-exo-2-[(endo-3-methylbicyclo[2.2.1]hept-endo-2-yl)methyl]bicyclo[2.2.1]heptane of formula (V), 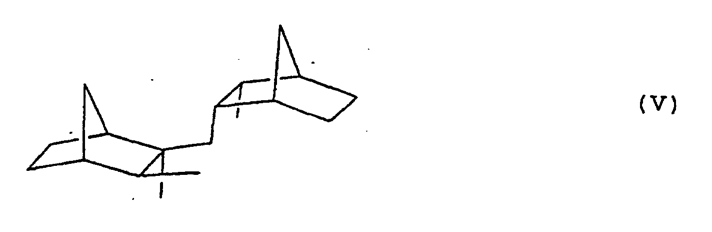 or endo-2-methyl-exo-3-methyl-exo-2-[(endo-2-methylbicyclo[2.2.1]hept-endo-3-yl)methyl]bicyclo[2.2.1]heptane of formula (VI) 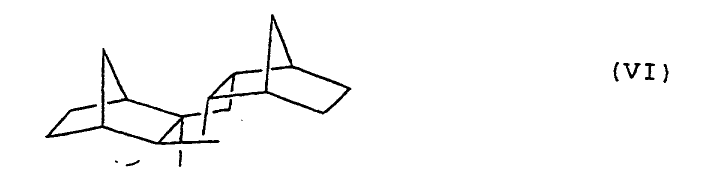
(2) A mixture of exo-2-methyl-exo-3-methyl-endo-2[(endo-3-methylbicyclo[2.2.1]hept-exo-2-yl)methyl]-bicyclo[2.2.1]heptane of formula II as defined in section (1) and exo-2-methyl-exo-3-methyl-endo-2-[(endo-2-methylbicyclo[2.2.1]hept-exo-3-yl)methyl]bicyclo[2.2.1]heptane of formula I as defined in section (1).
(3) A mixture of endo-2-methyl-exo-3-methyl-exo-2-[(exo-3-methyl-bicyclo[2.2.1]hept-exo-2-yl)methyl]-bicyclo[2.2.1]heptane of formula III as defined in section (1) and endo-2-methyl-exo-3-methyl-exo-2-[(exo-2-methylbicyclo[2.2.1]hept-exo-3-yl)-methyl]bicyclo[2.2.1]heptane of formula IV as defined in section (1).
(4) A mixture of endo-2-methyl-exo-3-methyl-exo-2-[(endo-3-methyl-bicyclo[2.2.1]hept-endo-2-yl)methyl] bicyclo[2.2.1]heptane of formula V as defined in section (1) and endo-2-methyl-exo-3-methyl-exo-2-[(endo-2-methylbicyclo[2.2.1]hept-endo-3-yl)methyl]bicyclo[2.2.1] heptane of formula VI as defined in section (1).
(5) A method for preparing a bicyclo[2.2.1]heptane derivative represented by any of the formulae (I) to (VI) in section (1), wherein a methylene- and methyl-substituted bicyclo[2.2.1]heptane ring compound and/ or a methyl-substituted bicyclo[2.2.1]heptene ring compound are dimerized in the presence of an acid catalyst at 60 °C or lower temperature and hydrogenating the produced dimer in the presence of a catalyst for hydrogenation at 200 - 300 °C .
(6) A method for preparing the bicyclo[2.2.1]heptane derivative according to section (5), wherein the methylene- and methyl-substituted bicyclo[2.2.1]heptane ring compound is 2-methylene-3-methylbicyclo [2.2.1]heptane and/or 3-methylene-2-methylbicyclo[2.2.1]heptane.
(7) A method for preparing a bicyclo[2.2.1]heptane derivative according to section (5), wherein the methyl-substituted bicyclo[2.2.1]heptene ring compound is 2,3-dimethylbicyclo[2.2.1] hept-2-ene.
(8) A method for preparing the bicyclo[2.2.1]heptane derivative according to section (5), wherein an acid catalyst is a Lewis acid.
(9) A method for preparing a bicyclo[2.2.1]heptane derivative according to section (5), wherein the catalyst for hydrogenation is a nickel catalyst.
(10) A fluid for a traction drive; characterized by consisting of a synthetic oil having physical properties:
   (i) Molecular weight: 210 or heavier.
   (ii) Kinematic viscosity at 40 °C : 10 - 25 mm²/s
   (iii) Viscosity index: 60 or higher
   ( iv) Pour point: -40 °C or lower
   (v) Density at 20 °C: 0.93 g/cm³ or higher
   (vi) Traction coefficient at 140 °C: 90% or higher of the coefficient of 2,4-dicyclohexyl-2-methylpentane and wherein said synthetic oil is at least one selected from the group consisting of the compounds according to formula (I) to (VI) defined in section (1).
(11) Fluid for a traction drive consisting of at least one compound selected from the group of compounds according to section (1).

### BRIEF DESCRIPTION OF THE DRAWINGS

Fig. 1. A mass chromatogram of component A.
Fig. 2. ¹H-NMR spectrogram of component A.
Fig. 3. ¹³C-NMR spectrogram of component A.
Fig. 4. ¹³C-¹³C two-dimensional NMR spectrogram of component A.
Fig. 5. ¹H-¹³C two-dimensional NMR spectrogram of component A.
Fig. 6. A mass chromatogram of component B.
Fig. 7. ¹H-NMR spectrogram of component B.
Fig. 8. ¹³C-NMR spectrogram of component B.
Fig. 9. ¹³C-¹³C two-dimensional NMR spectrogram of component B.
Fig. 10. ¹H-¹³C two-dimensional NMR spectrogram of component B.
Fig. 11. A mass chromatogram of component C.
Fig. 12. ¹H-NMR spectrogram of component C.
Fig. 13. ¹³C-NMR spectrogram of component C.
Fig. 14. ¹³C-¹³C two-dimensional NMR spectrogram of component C.
Fig. 15. ¹H-¹³C two-dimensional NMR spectrogram of component C.

### DETAILED DESCRIPTION OF THE PREFERRED EMBODIMENTS

Below will be fully described the invention.

### 1. A derivative of bicyclo[2.2.1]heptane

The present invention is a derivative of bicyclo[2.2.1] heptane with a special structure, exemplified by bicyclo[2.2.1]heptane, exo-2-methyl-exo-3-methyl-endo-2-[(endo-2-methylbicyclo[2.2.1]hept-exo-3-yl)methyl] bicyclo[2.2.1]heptane, exo-2-methyl-exo-3-methyl-endo-2-[(endo-3-methylbicyclo[2.2.1]hept-exo-2-yl)methyl] bicyclo[2.2.1]heptane, endo-2-methyl-exo-3-methyl-exo-2-[(exo-3-methylbicyclo-[2.2.1]hept-exo-2-yl)methyl] bicyclo[2.2.1]heptane, endo-2-methyl-exo-3-metyl-exo-2-[(exo-2-methyl bicyclo[2.2.1]hept-exo-3-yl)methyl] bicyclo[2.2.1]heptane, endo-2-methyl-exo-3-methyl-exo-2-[(endo-3-methylbicyclo[2.2.1]hept-endo-2-yl)methyl] bicyclo[2.2.1]heptane, or endo-2-methyl-exo-3-methylexo-2-[(endo-2-methylbicyclo[2.2.1]hept-endo-3-yl) methyl]bicyclo[2.2.1]heptane.

These compounds are separately represented by one of the following chemical structural formula (I) to the formula (VI).

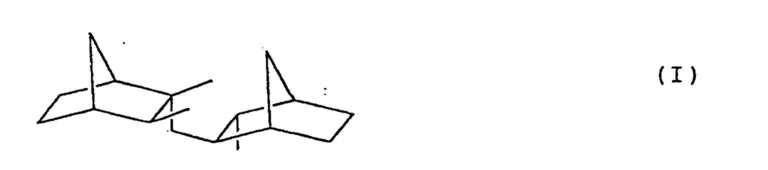

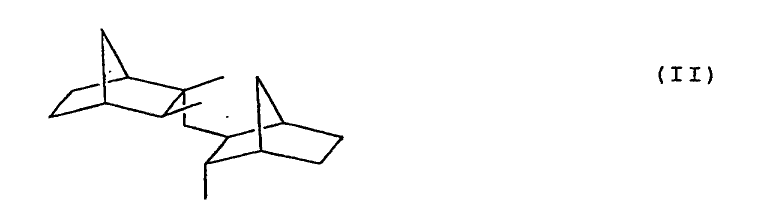

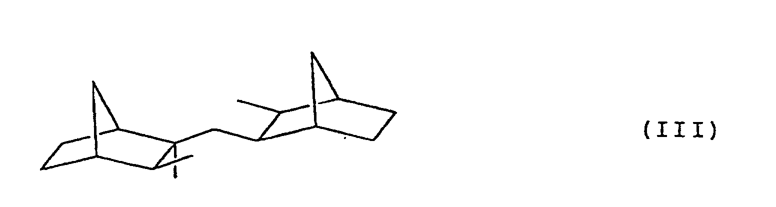

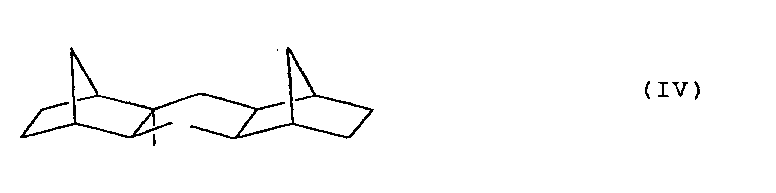

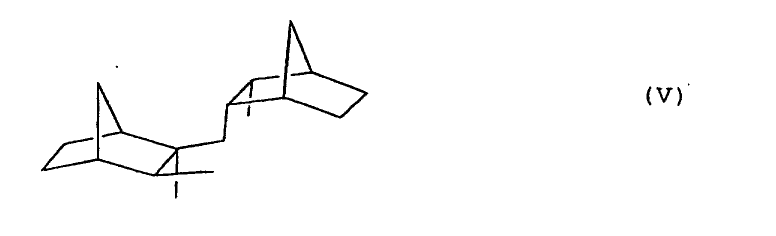

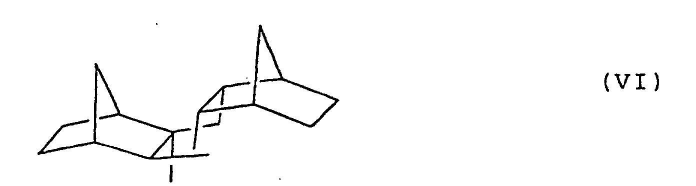

These bicyclo[2.2.1]heptane derivatives have particularly high traction coefficient under high temperature and an excellent characteristic of viscosity under low temperature to allow preferably their use as a CVT oil for a traction drive in the world from cold and tropical regions and in addition, are compounds having very high 'value for use for the purpose of decreasing the CVT for the traction drive.

These compounds (I) to (VI) are included in bicyclo[2.2.1]heptane derivatives represented by the formula (VII) and are prepared by the same method as production method of bicyclo [2.2.1] heptane derivatives represented by the following described formula (VII) 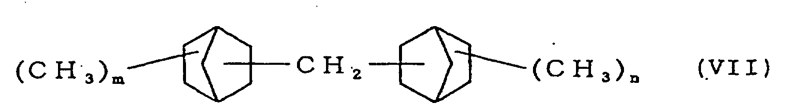 wherein m represents 2 or 3 and n represents 1 or 2, using a material compound suitably selected. The chemical structure of these compounds is determined by analysis of each spectrogram of mass chromatogram, ¹H-NMR, ¹³C-NMR, ¹³C-¹³C-NMR, and ¹H-¹³C-NMR.

### 2. A method for producing bicyclo[2.2.1]heptane derivatives

The invention is a method for producing bicyclo[2.2.1] heptane derivatives represented by any of the formulae (I) to (VI) by dimerization of materials that are methylene group and bicyclo[2.2.1]heptane ring compounds with a substituted methyl group and/or bicyclo[2.2.1]heptene ring compounds with a substituted methyl group under 60 °C in the presence of an acid catalyst and hydrogenating the produced dimer under 200 - 300 °C in the presence of a hydrogenating catalyst.

In the formula, the methylene group and bicyclo[2.2.1]heptane ring compounds, substituted methylene group and with methyl group, are exemplified by 2-methylene-3-methylbicyclo[2.2.1]heptane, 3-methylene-2-methylbicyclo[2.2.1]heptane, 2-methylene-7-methylbicyclo[2.2.1]heptane, 3-methylene-7-methyl bicyclo[2.2.1]heptane, 2-methylene-5-methylbicyclo[2.2. 1]heptane, 3-methylene-5-methylbicyclo[2.2.1]heptane, 2-methylene-6-methylbicyclo[2.2.1]heptane, 3-methylene-6-methylbicyclo[2.2.1]heptane, 2-methylene-1-methylbicyclo[2.2.1]heptane, 3-methylene-1-methyl bicyclo[2.2.1]heptane, 2-methylene-4-methylbicyclo [2.2.1]heptane, 3-methylene-4-methylbicyclo[2.2.1] heptane, 2-methylene-3,7-dimethylbicyclo[2.2.1]heptane, 3-methylene-2,7-dimethylbicyclo[2.2.1]heptane, 2-methylene-3,6-dimethylbicyclo[2.2.1]heptane, 3-methylene-2,6-dimethylbicyclo[2.2.1]heptane, 2-methylene-3,3-dimethylbicyclo[2.2.1]heptane, 3-methylene-2,2-dimethylbicyclo[2.2.1]heptane.

Among them, 2-methylene-3-methylbicyclo[2.2.1] heptane is preferable in the presence of exo-3-methyl-2-methylenebicyclo[2.2.1]heptane and endo-3-methyl-2-methylenebicyclo[2.2.1]heptane and 3-methylene-2-methyl bicyclo[2.2.1]heptane in the presence of exo-2-methyl-3-methylenebicyclo[2.2.1]heptane and endo-2-methyl-3-methylenebicyclo[2.2.1]heptane.

On the other hand, the bicyclo[2.2.1]heptene ring compounds with substituted methyl group are exemplified by 2,3-dimethylbicyclo[2.2.1]hept-2-ene, 2,7-dimethyl bicyclo[2.2.1]hept-2-ene, 2,5-dimethylbicyclo[2.2.1] hepto-2-ene, 2, 6-dimethylbicyclo[2.2.1]hept-2-ene, 1,2-dimethylbicyclo[2.2.1]hept-2-ene, 2,4-dimethyl bicyclo[2.2.1]hept-2-ene, 2,3,7-trimethylbicyclo [2.2.1]hept-2-ene, 2,3,6-trimethylbicyclo [2.2.1] hept-2-ene, etc.

Among them, 2,3-dimethylbicyclo[2.2.1]hept-2-ene is preferable.

For the purpose of producing bicyclo[2.2.1]heptane derivatives having the stereochemical structure represented by said formulae from (I) to (VI), the mixture .of 2-methylene-3-methylbicyclo[2.2.1]heptane, 3-methylene-2-methylbicyclo[2.2.1]heptane, and 2,3-dimethylbicyclo[2.2.1]hept-2-ene is preferably used as material olefin.

In the invention, said material olefin is first dimerized in the presence of an acid catalyst. A compound, either inorganic acid or organic acid, showing acid property can be used as the acid catalyst. Besides, concerning the form of the acid catalyst, either liquid or solid can be used. Preferable acid catalysts are exemplified by hydrofluoric acid, mineral acids such as polyphosphoric acids, organic acids such as triflic acid, Lewis acids such as aluminium chloride, ferric chloride, tin tetrachloride, titanium tetrachloride, boron trifluoride, a complex of boron trifluoride, boron tribromide, aluminium bromide, gallium chloride, and gallium bromide, and organic aluminium compounds such as triethyl aluminium, diethyl aluminium chloride, and diethyl aluminium dichloride, etc.

Among these acid catalysts, such Lewis acids as boron trifluoride, a complex of boron trifluoride, tin tetrachloride, titanium tetrachloride, and aluminium chloride. Among them, such complexes of boron trifluoride as diethyl ether complex of boric trifluoride, water complex of boron trifluoride, and alcohol complex of boron trifluoride are further preferable in the point of possible dimerization under lower temperature.

The production of bicyclo[2.2.1]heptane derivatives having a stereochemical structure represented by said formulae from (I) to (VI) can be preferably carried out by using a diethyl ether complex of boron trifluoride as catalyst for dimerization.

Concerning the amounts used for these catalysts, there is no special restriction, however, usually, 0.1 - 100 weight percent in proportion to material olefin, preferably around 0.5 - 20 weight percent.

In the dimerization reaction there is not always used a solvent, however, the solvent may be used for handling of material olefin at reaction time or regulating the progress of the reaction. Preferable solvents are exemplified by saturated hydrocarbons such as various pentanes, various hexanes, various octanes, various nonanes, various decanes, etc., alicyclic hydrocarbons such as cyclopentane, cyclohexane, methylcyclosane, decalin, etc., ether compounds such as diethyl ether, tetrahydrofuran, etc., compounds containing halogens such as methylene chloride, dichloroethane, etc., and nitro compounds such as nitromethane, nitrobenzene, etc.

The dimerization reaction is usually carried out under a reaction temperature of 60 °C or lower, preferably 40 °C or lower. A reaction temperature over 60 °C allows isomerization of the dimer produced by the reaction. The target bicyclo[2.2.1]heptane derivative of a stereochemical structure is preferably yielded with a high purity by the reaction of dimerization under lower temperature, because a plurality of stereochemical isomers produced by isomerization reaction is difficult to separate. The lower limit of the temperature range is not restricted, when the dimerization reaction is going well; however, -70 °C or higher temperature is economically preferable and -30°C or higher temperature is further preferable.

The reaction pressure of dimerization reaction is determined to an appropriate range according to reaction temperature, kind of catalyst, presence or absence of solvent, and kind of solvent; however, an ordinary pressure is usually preferable. In addition, reaction time is preferably in the range of 0.5 - 10 hours usually.

In the invention, the dimer of the material olefin obtained by such conditions is hydrogenated in the presence of a catalyst for hydrogenation.

Support of the catalyst for hydrogenation usually compounded with a metal component can be used. A preferred example is a catalyst in which a metal component of an element such as nickel, ruthenium, palladium, platinum, rhodium, and iridium of VIII group of the periodic table is contained in inorganic oxide support such as diatomite, alumina, silica alumina, and activated clay. Among them, nickel catalyst such as nickel/diatomite, nickel/silica alumina, etc. is preferable in the point of economy and possible selection of product. Besides, such solid acids as zeolite, silica alumina, and activated clay as a promoter for hydrogenation may be used, if necessary.

For reference, the production of bicyclo[2.2.1]heptane derivatives having a stereochemical structure that is represented by the formulae from (I) to (VI) is preferably carried out using nickel/diatomite catalyst as catalyst for hydrogenation.

The amount of catalyst used is usually 0.1 - 100 weight percent in the proportion to said dimerized product, preferably 1 - 20 weight percent.

On the other hand, hydrogenation reaction goes without any solvent just as said dimerization reaction; however, solvent is preferably used and a preferable solvent is exemplified by saturated hydrocarbons such as various pentanes, various hexanes, various octanes, various nonanes, various decanes, etc., alicyclic hydrocarbons such as cyclopentane, cyclohexane, methylcyclosane, decalin, etc.

The reaction temperature for the hydrogenation reaction is preferably 200 - 300 °C and further preferably 220 - 280 °C. A reaction temperature lower than 200 °C may insufficiently decrease the viscosity of the product by isomerization and may insufficiently.increase the viscosity index. On the other hand, a temperature higher than 300 °C may lower the yield by decomposition of the product.

Furthermore, the reaction pressure is not specially restricted usually to allow an ordinary pressure to 19.6 MPaG (200 kg/cm² G), preferably an ordinary pressure to 9.8 MPa G (100 kg/cm² G). Hydrogen pressure is preferably in 0.49-8.82 MPaG (5 - 90 kg/cm² G), further preferably 0.98-7.84 MPaG (10 - 80 kg/cm² G). Reaction time is usually 1 - 10 hours.

Bicyclo[2.2.1]heptane derivatives represented by said formulae (I) to (VI) and yielded under such conditions show a high traction coefficient under high temperature and an excellent characteristic of viscosity under low temperature to allow their use as a CVT oil for traction drive in cold as well as tropical regions.

### 3. Fluid for traction drive

The fluid for traction drive of the invention was obtained on the basis of a finding that bicyclo[2.2.1] heptane derivatives, particularly bicyclo[2.2.1]heptane derivatives having a stereochemical structure represented by the formulae from (I) to (IV), have excellent physical properties as the f luid for traction drive.

The fluid for traction drive of the invention is consisting of a synthetic oil having physical properties of the following (1) to (6).
(1) The number-average molecular weight of a compound contained in a synthetic oil is 210 or more, preferably 215 ∼ 290. The number-average molecular weight less than 210 may cause an increase in the volatilized amount of the fluid for traction drive in the use under high temperature.
(2) The kinematic viscosity of a synthetic oil at 40 °C is 10 - 25 mm²/s, preferably 12 - 24 mm²/s. A kinematic viscosity of synthetic oil at 40 °C of less than 10 mm²/s may cause low viscosity under high temperature to result in an insufficient thickness of the layer of lubricating oil. On the other hand, a kinematic viscosity which exceeds 25 mm²/s at 40 °C may cause the low temperature viscosity exceeding 150000 mPa·s under -40 °C.
(3) The viscosity index of synthetic oil is 60 or higher, preferably 65 or higher. An index of less than 60 may cause a low temperature viscosity exceeding 150 000 mPa.s under -40 °C.
(4) Pour point of synthetic oil is under -40 °C, preferably under -45 °C. A pour point higher than -40 °C causes difficulty of starting the traction drive machine in use in a cold region.
(5) The density of synthetic oil under 20 °C is higher than 0.93 g/cm³, preferably in the range from 0.93 to 1.50 g/cm³. A density less than 0.93 g/cm³ is not preferable due to the occurrence of lower traction coefficient under 140 °C.
(6) The traction coefficient of synthetic oil under the temperature of 140 °C is 90% or higher of that of 2,4-dicyclohexyl-2-methylpentane, preferably that of 2,4-dicyclohexyl-2-methylpentane. A coefficient less than 90% of that of 2,4-dicyclohexyl-2-methylpentane may cause insufficient capacity of torque transmission under 140 °C.

The synthetic oil of the invention meeting the physical properties of said (1) to (6) is selected from the compounds of formulae (I) to (VI) defined above.

All compounds of formula (I) to (VI) are generally a hydrogenated dimer of bicyclo[2.2.1]heptane ring compounds represented by the following formula (VIII). 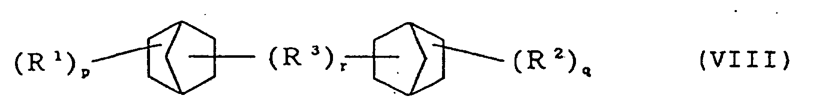 (In the formula, both R¹ and R² represent hydrogen atoms or alkyl groups with carbon numbers of 1 - 3, R³ represents methylene group, ethylene group, or trimethylene group that may be substituted by methyl group or ethyl group in a side chain, r represents 0 or 1, both p and q represent integral numbers of 1 - 3).

More specifically, they belong to the bicyclo[2.2.1]heptane derivatives represented by the following formula (VII),

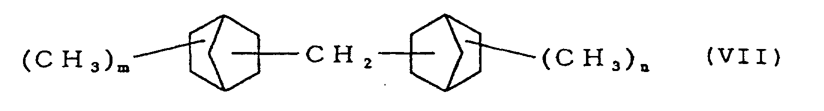

Bicyclo[2.2.1] heptane derivatives having a stereochemical structure represented by the following formula (I) to (VI) are the synthetic oil of the fluid for a traction drive according to the invention.

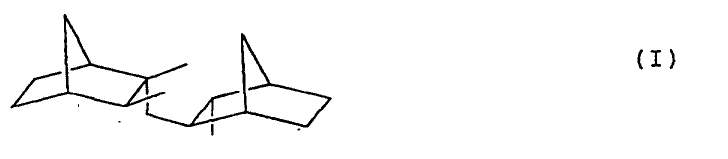

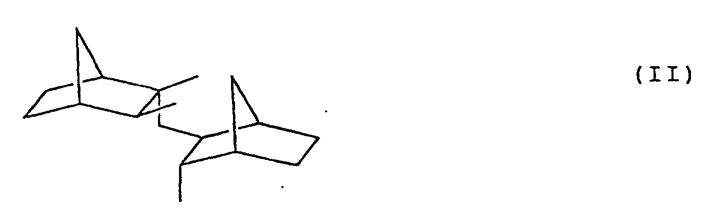

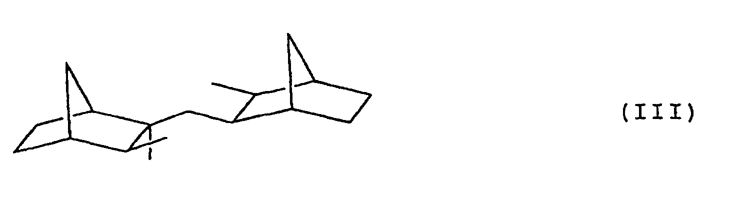

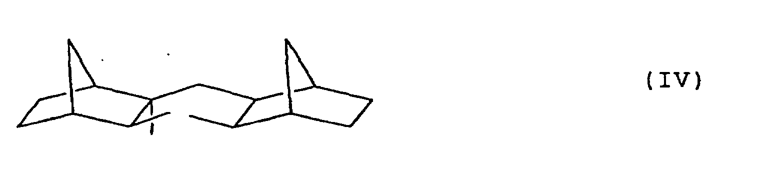

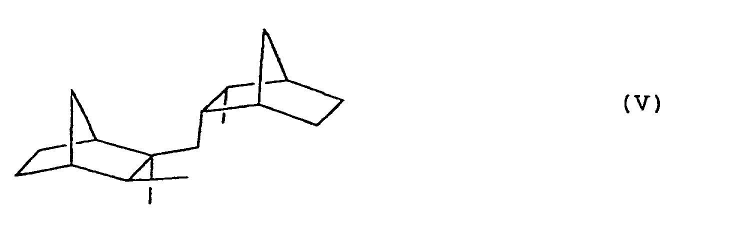

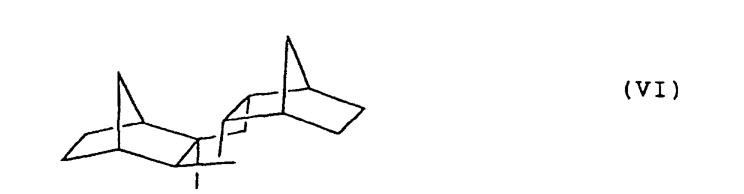

(In the formula, m represents 2 or 3 and n represents 1 or 2).

Said hydrogenated compounds can be used as a single compound or a mixture of two or more compounds. Hydrogenated compounds represented by said formulae (VIII) can be prepared by dimerization, hydrogenating, and distillation of the material olefinic alicyclic compound.

The material olefinic alicyclic compound is exemplified by bicyclo[2.2.1]hept-2-ene, alkenyl-substituted bicyclo[2.2.1]hept-2-ene such as vinyl-substituted or isopropenyl-substituted bicyclo[2.2.1]hept-2-ene, alkylidene-substituted bicyclo[2.2.1]hept-2-ene such as methylene-substituted, ethylidene-substituted, or isopropylidene-substituted bicyclo[2.2.1]hept-2-ene, alkenyl-substituted bicyclo[2.2.1]heptane such as vinyl-substituted or isopropenyl-substituted bicyclo [2.2.1]heptane, alkylidene-substituted bicyclo[2.2.1] heptane such as methylene-substituted, ethylidene-substituted or isopropylidene-substituted bicyclo [2.2.1]heptane, bicyclo[3.2.1]octene, alkenyl-substituted bicyclo[3.2.1]octene such as vinyl-substituted or isopropenyl-substituted bicyclo[3.2.1] octene, alkylidene-substituted bicyclo[3.2.1]octene such as methylene-substituted, ethylidene-substituted, or isopropylidene-substituted bicyclo[3.2.1]octene, alkenyl-substituted bicyclo[3.2.1]octane such as vinyl-substituted or isopropenyl-substituted bicyclo [3.2.1]octane, alkylidene-substituted bicyclo [3.2.1) octane such as methylene-substituted, ethylidene-substituted, or isopropylidene-substituted bicyclo [3.2.1]octane, bicyclo[3.3.0]octene, alkenyl-substituted bicyclo[3.3.0]octene such as vinyl-substituted or isopropenyl-substituted bicyclo[3.3.0] octene, alkylidene-substituted bicyclo[3.3.0]octene such as methylene-substituted, ethylidene-substituted, or isopropylidene-substituted bicyclo[3.3.0]octene, alkenyl-substituted bicyclo[3.3.0]octane such as vinyl-substituted or isopropenyl-substituted bicyclo [3.3.0]octane, alkylidene-substituted bicyclo[3.3.0] octane such as methylene-substituted, ethylidene-substituted, or isopropylidene-substituted bicyclo [3.3.0]octane, bicyclo[2.2.2]octene, alkenyl-substituted bicyclo[2.2.2]octene such as vinyl-substituted or isopropenyl-substituted bicyclo[2.2.2] octene, alkylidene-substituted bicyclo[2.2.2]octene such as methylene-substituted, ethylidene-substituted, or isopropylidene-substituted bicyclo[2.2.2]octene, alkenyl-substituted bicyclo [2.2.2] octane such as vinyl-substituted or isopropenyl-substituted bicyclo [2.2.2] octane, alkylidene-substituted bicyclo [2.2.2] octane such as methylene-substituted, ethylidene-substituted, or isopropylidene-substituted bicyclo [2.2.2]octane.

Among them, appropriate material alicyclic compounds, represented by said formula (VIII), are exemplified by bicyclo [2.2.1]hept-2-ene; 2-methylenebicyclo[2.2.1]heptane; 2-methylbicyclo[2.2.1]hept-2-ene; 2-methylene-3-methylbicyclo[2.2.1]heptane; 2,3-dimethylbicyclo [2.2.1]hept-2-ene; 2-methylene-7-methylbicyclo[2.2.1] heptane; 2.7-dimethylbicyclo[2.2.1]hept-2-ene; 2-methylene-5-methylbicyclo[2.2.1]heptane; 2,5-dimethyl bicyclo[2.2.1] hept-2-ene; 2-methylene-6-methylbicyclo [2.2.1]heptane; 2,6-dimethylbicyclo[2.2.1]hept-2-ene; 2-methyl-1-methylbicyclo[2.2.1]heptane; 1,2-dimethyl bicyclo[2.2.1]hept-2-ene; 2-methylene-4- methylbicyclo [2.2.1]heptane; 2,4-dimethylbicyclo [2.2.1] hept-2-ene; 2-methylene-3.7-dimethylbicyclo[2.2.1]heptane; 2,3,7-trimethylbicyclo[2.2.1]hept-2-ene; 2-methylene-3.6-dimethylbicyclo[2.2.1]heptane; 2-methylene-3.3-dimethylbicyclo[2.2.1]heptane; 2,3,6-trimethylbicyclo [2.2.1]hept-2-ene; 2-methylene-3-ethylbicyclo[2.2.1] heptane; 2-methyl-3-ethylbicyclo[2.2.1]hept-2-ene.

Said dimerization means not only the dimerization of same olefins, but also the dimerization of plural different olefins.

The dimerization reaction of said olefinic alicyclic ring compounds is carried out by adding solvent, if necessary, in the presence of catalyst.

The catalyst used for the dimerization reaction is, usually, acid catalyst. Individual catalyst can be same as that used for the production of bicyclo[2.2.1]heptane derivatives represented by said following formula (VII) and its detailed description is omitted here. In addition, the amount of used catalyst and the condition of reaction are also same and its detailed description is omitted here.

In the invention, the dimer yielded is hydrogenated. The condition of hydrogenating treatment and other conditions are same as those used for the production of bicyclo[2.2.1]heptane derivatives represented by said formula (VII) and its detailed description is omitted here.

In the invention, the synthetic oil having physical properties of said (1) - (6) and being at least one of the compounds of formula (I) to formula (VI) is used for base oil of the fluid for traction drive, however, other liquid matter may be mixed with the synthetic oil for use in the range within the range of the physical properties of said (1) - (6). For example, such base oil with low viscosity as poly α-olefin oil (PAO) and diester can be mixed to keep a traction coefficient under high temperature and such base material as the base oil of alicyclic compound for traction and hydrogenated dicyclopentadiene petroleum resin, etc., to keep a viscosity under low temperature can be mixed to improve a traction coefficient under a high temperature. The mixing amount for addition of other liquid matter is preferably 15 weight percent or less.

In the use for the fluid for traction drive, the base oil with said synthetic oil as a main component is compounded with such additives as antioxidant, rust inhibitor, detergent dispersant, pour point depressant, an viscosity index improver, extreme pressure agent, anti-water agent, oiliness agent, antifoaming agent, corrosion inhibitor, etc., in suitable amount if necessary.

### Examples

Examples of the invention are separately given below Example 1.

### Production of material olefin

Crotonaldehyde (561 g = 8 mol) and bicyclopentadiene (352 g = 2.67 mol) were put in 2 L, stainless steel-made autoclave to react by mixing for 3 hours under 170 °C. The reaction solution was cooled to room temperature, 18 g of a Raney nickel catalyst (Kawaken Fine Chemicals, K.K. made, M-300T) was added, and finally, hydrogenation was carried out under a hydrogen pressure of 0.88 MPaG (9 kg/ cm2 G) and reaction temperature of 150 °C for 4 hours. After cooling, the catalyst was filtered to separate, the filtrate was distilled under a reduced pressure to yield 565 g of fraction under 105°C/2.66 kPa (20 mm Hg). The result of mass spectrum analysis and nuclear magnetic resonance spectrum analysis of the fraction showed that the fraction is 2- hydroxy methyl-3-methylbicyclo[2.2.1]heptane and 3-hydroxy methyl-2-methylbicyclo[2.2.1]heptane.

Next, 20 g of γ-alumina [Nikki Kagaku, K.K. made, N612N] was put in a quartz glass-made flow reaction tube under an atmospheric pressure, with an outer diameter of 20 mm and a length of 500 mm to start dehydration reaction going under a reaction temperature of 285 °C and weight hourly space velocity (WHSV) = 1.1 hr⁻¹ and finally, 450 g of the dehydrated reaction product of 2-hydroxymethyl-3-methylbicyclo[2.2.1]heptane and 3-hydroxymethyl-2-methylbicycio[2.2.1]heptane was yielded containing 55 weight percent of 2-methylene-3-methylbicyclo[2.2.1] heptane and 3-methylene-2-methylbicyclo[2.2.1]heptane and 30 weight percent of 2,3-dimethylbicyclo[2.2.1] hept-2-ene.

### Production of hydrogenated dimer

Diethyl ether complex (8 g) of boron trifluoride and the olefin compound (400 g) yielded from said steps were put in a 1-L four-neck flask and stirred under 20 °C for 4 hours by using a mechanical stirrer to start the dimerization reaction going. The reaction mixture was washed with diluted NaOH aqueous solution and saturated brine, 12 g of nickel/diatomite catalyst [Nikki Kagaku, K.K. made, N-113] for hydrogenation was added in a 1-L autoclave, and finally, hydrogenating reaction was started under the condition of 2.94 MPaG (30 kg/cm² G) of hydrogen pressure, reaction temperature of 250 °C, and reaction time of 6 hours. After the completion of the reaction, the catalyst was removed by filtration and the filtrate was distilled under a reduced pressure to yield 240 g of a target hydrogenated dimer. Table 1 shows the measured results of general properties and the traction coefficient of the hydrogenated dimer.

Twice rectification to separate the hydrogenated dimer was carried out to yield 1 g of a component under 149.2 °C 667 Pa (5 mm Hg) using a spinning band distillation apparatus with 120 theoretical plate number. The analysis of the component gave exo-2-methyl-exo-3-methyl-endo-2-[(endo-3-methyl bicyclo [2.2.1] hept-exo-2-yl) methyl] bicyclo [2.2.1]heptane (the compound of said formula (II)) and exo-2-methyl-exo-3-methyl-endo-2- [(endo-2-methyl bicyclo[2.2.1]hept-exo-3-yl)methyl]bicyclo[2.2.1] heptane (the compound of said formula (I)) (hereafter, both these compounds are called component A) with purity of 98 weight percent.

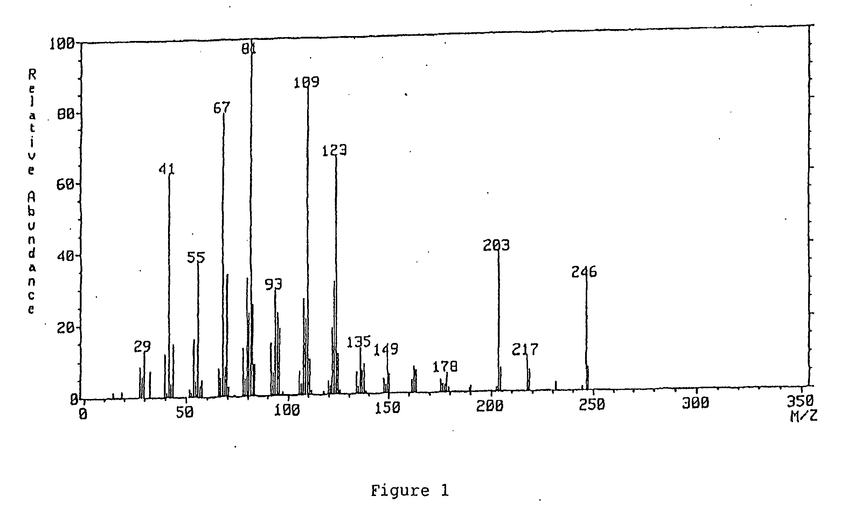

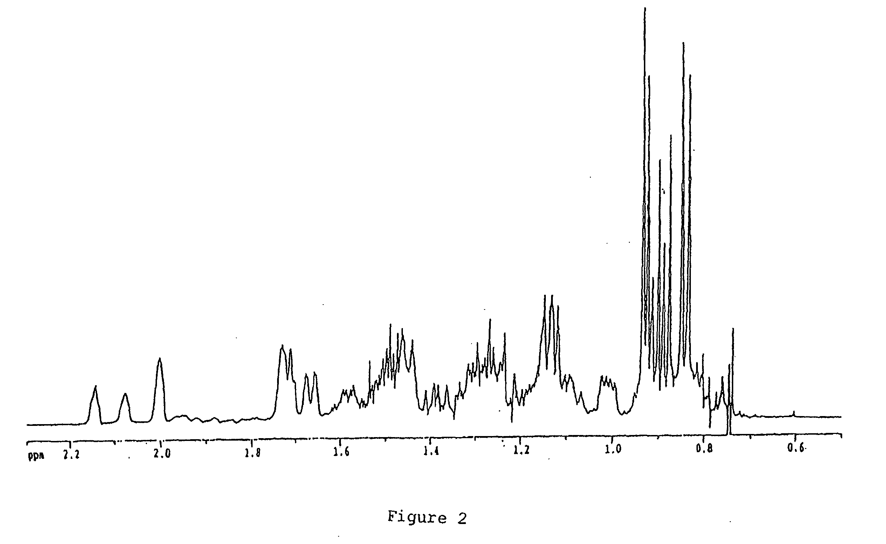

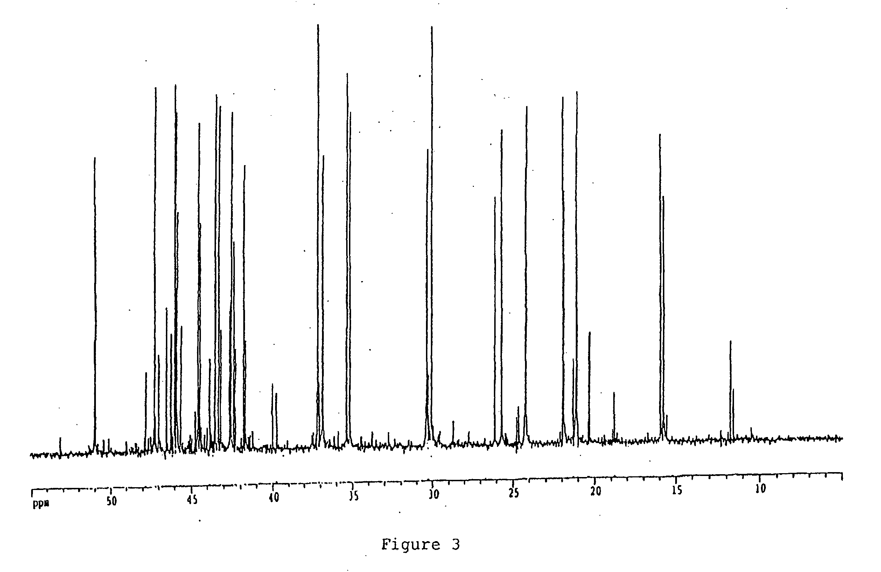

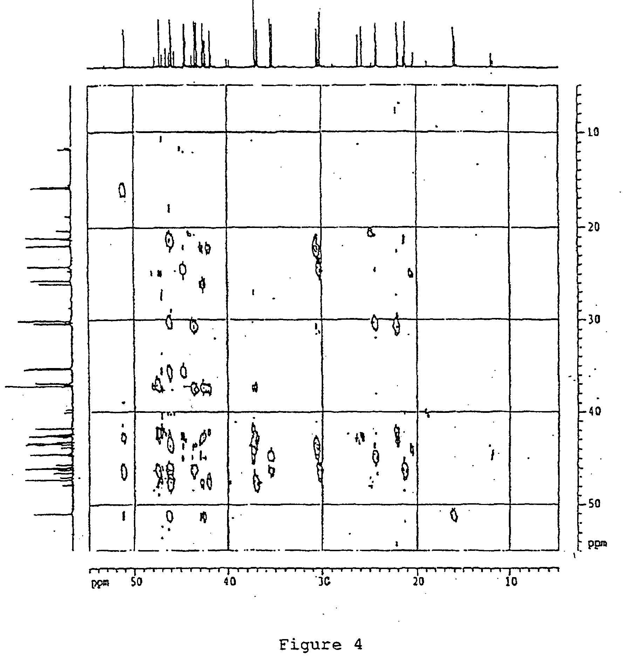

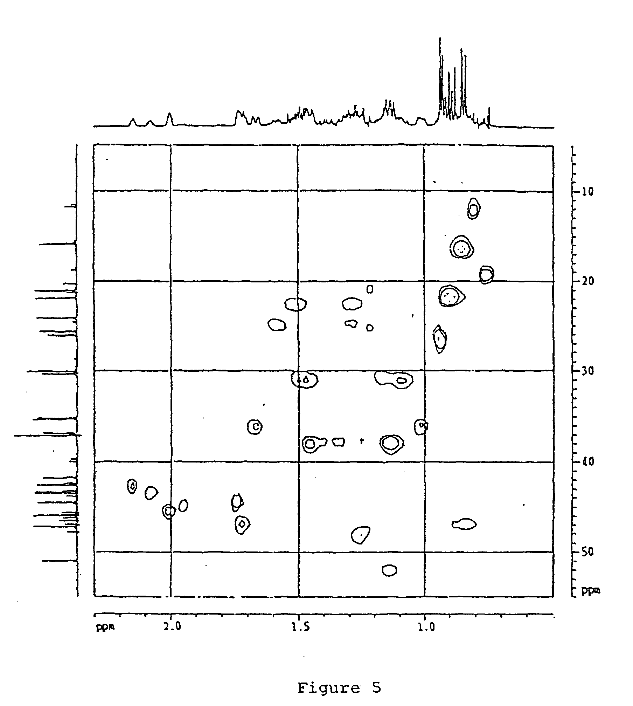

Figs 1 - 5 show each spectrogram of mass chromatogram, ¹H-NMR, ¹³C-NMR, ¹³C-¹³C-NMR, and ¹H-¹³C-NMR used for structure analysis of the component A.

Besides, rectification for separation same as above yielded 1 g of a component under 133.6 °C/266 Pa (2 mmHg). The analysis of the component showed endo-2-methyl-exo-3-methyl-exo-2-[(exo-3-methylbicyclo-[2.2.1]hept-exo-2-yl)methyl]bicyclo[2.2.1]heptane (the compound of said formula (III)) and endo-2-methyl-exo-3-metyl-exo-2-[(exo-2-methylbicyclo[2.2.1]hepto-exo-3-yl)methyl] bicyclo[2.2.1]heptane (the compound of said formula (IV)) (hereafter, both these compounds are called component B) with purity of 99 weight percent.

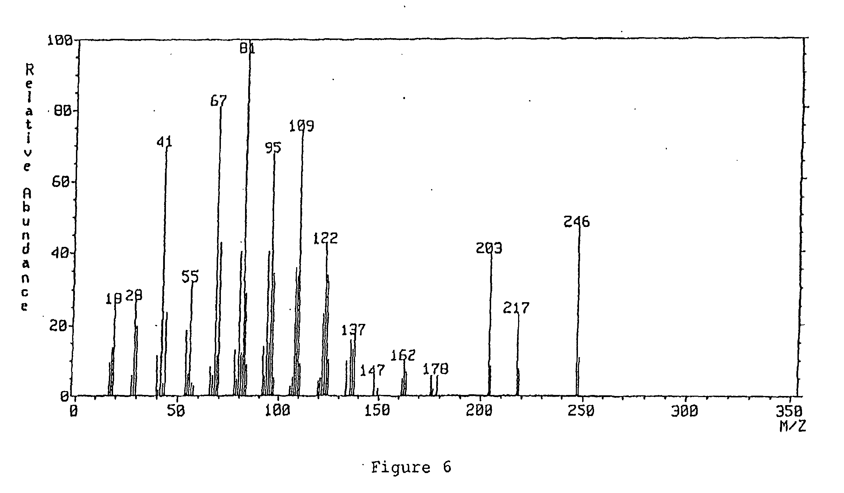

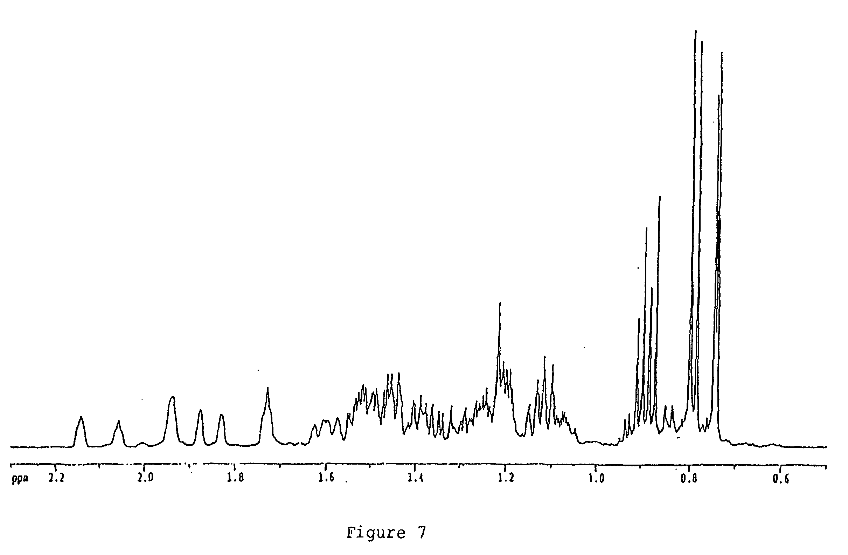

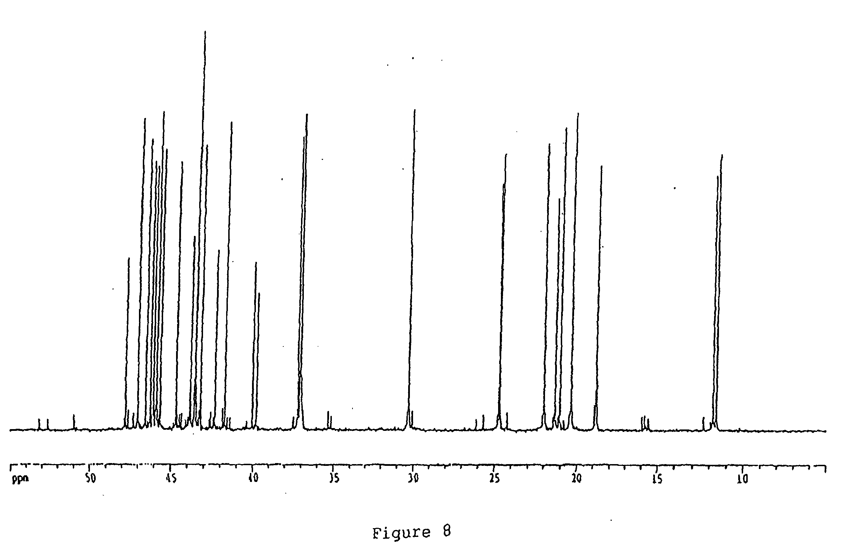

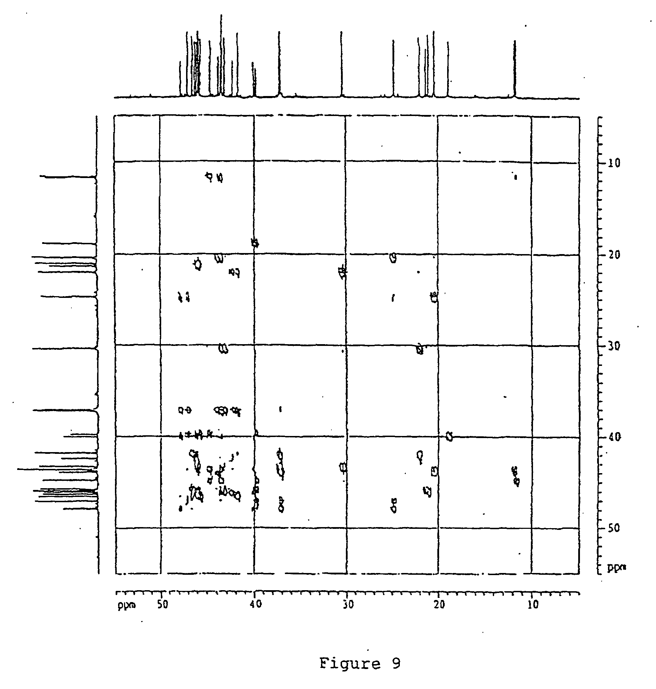

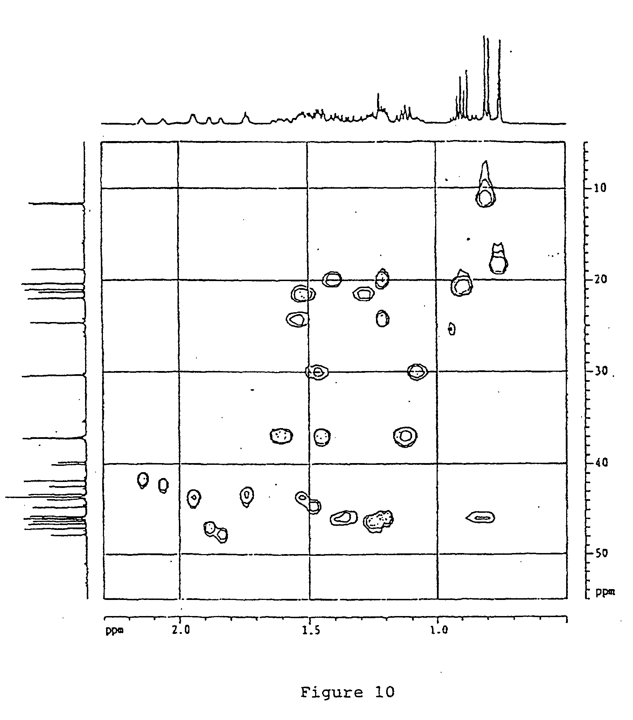

Figs 6 - 10 show each spectrogram of mass chromatogram, ¹H-NMR, ¹³C-NMR, ¹³C-¹³C-NMR, and ¹H-¹³C-NMR used for structure analysis of the component B.

The result of analysis of the hydrogenated dimer prepared in the Example 1 with gas chromatography showed contents of 20 weight percent of the component A and 60 weight percent of the component B.

### Example 2

Table 1 shows the measured result of general properties and a traction coefficient of the fraction, that contains 65 weight percent of the component A and 25 weight percent of the component B, yielded by the rectification for separation of the Example 1.

### Example 3

The hydrogenated dimer of 240 g was yielded by changing the hydrogenating reaction of Example 1 under the condition of 250 °C and 6 hours to that under 200 °C and 2 hours. Twice rectification to separate the hydrogenated dimer was carried out to yield 1 g of said component B using a spinning band distillation apparatus with 120 theoretical plate number.

Besides, rectification for separation same as above yielded 1 g of a component under 138.6 °C/266 Pa (2 mmHg). The analysis of the component showed endo-2-methyl-exo-3-methyl-exo-2-[(endo-3-methylbicycle-[2.2.1]hept-endo-2-yl)methyl]bicyclo[2.2.1]heptane (the compound of said formula (V)) and endo-2-methyl-exo-3-metyl-exo-2-[(endo-2-methylbicycle[2.2.1]hept-endo-3-yl)methyl] bicyclo[2.2.1]heptane (the compound of said formula (VI)) (hereafter, both these compounds are called component C) with purity of 100 weight percent.

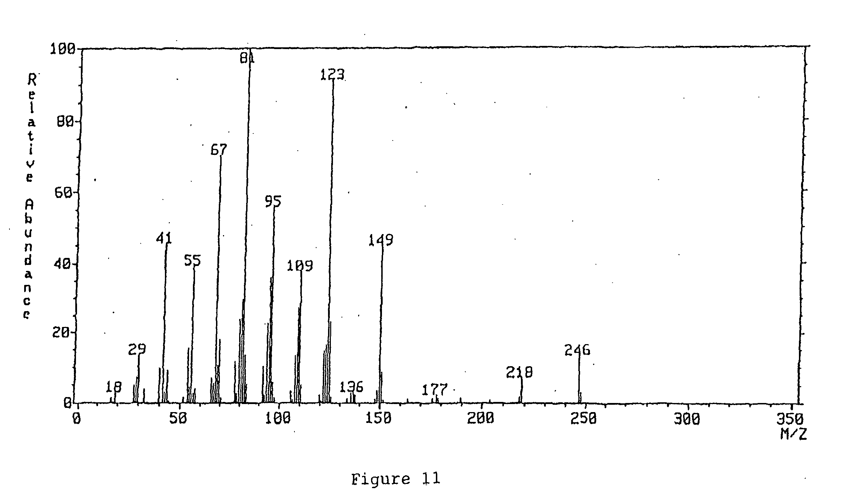

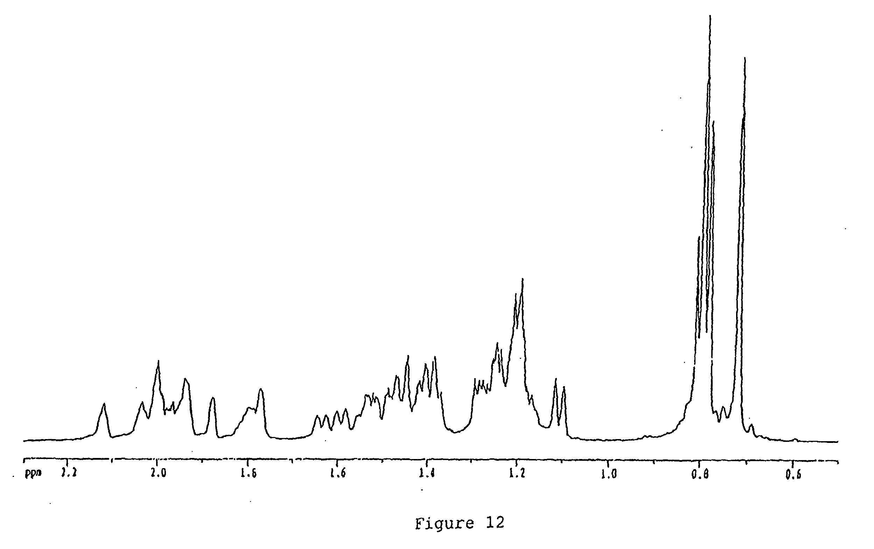

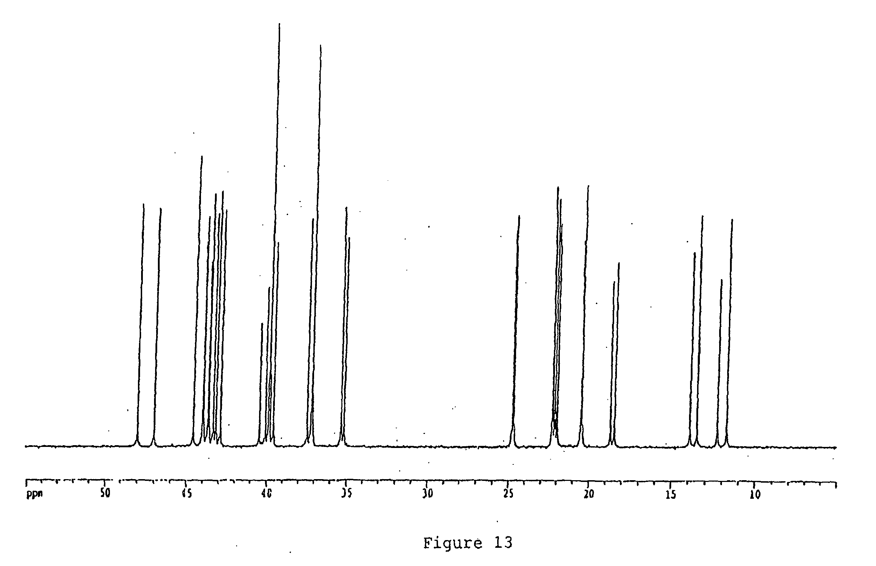

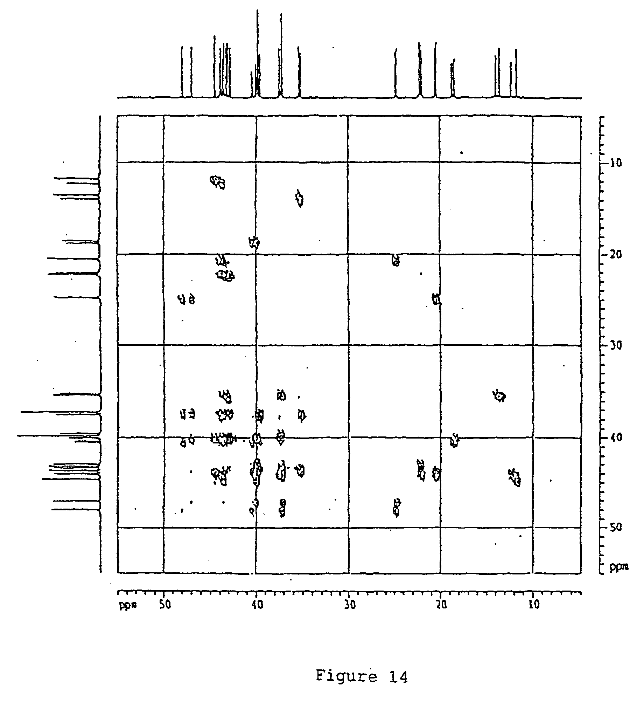

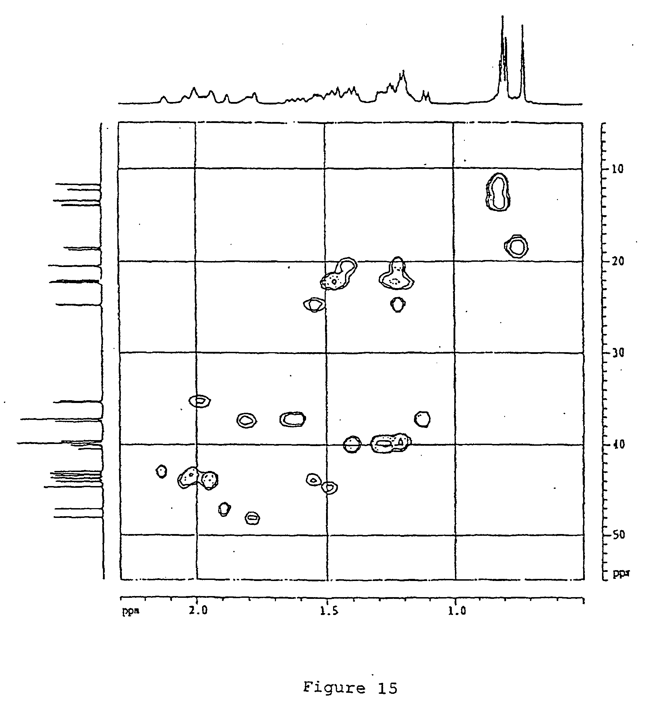

Figs 11 - 15 show each spectrogram of mass chromatogram, ¹H-NMR, ¹³C-NMR, ¹³C-¹³C-NMR, and ¹H-¹³C-NMR used for the structural analysis.

The result of the analysis of the hydrogenated dimer prepared in the Example 3 with gas chromatography showed contents of 45 weight percent of the component B and 45 weight percent of the component C.

### Example 4

Table 1 shows the measured result of general properties and a traction coefficient of the fraction, that contains 88 weight percent of the component B and 10 weight percent of the component C, yielded by the rectification for .separation of the Example 3.

### Example 5

140 g of hydrogenated dimer was yielded by replacing the catalyst of 8 g of diethyl ether complex of boron trifluoride for dimerization of the Example 1 to 32 g of tin tetrachloride. The result of the analysis of the hydrogenated dimer with gas chromatography showed contents of 20 weight percent of the component A and 60 weight percent of the component B. Table 1 shows the result of measurement of general properties and the traction coefficient of the fraction.

### Example 6 (Reference Example)

280 g of hydrogenated dimer was yielded by replacing the catalyst of 8 g of diethyl ether complex of boron trifluoride for dimerization to 20 g of 116% polyphosphoric acid by reaction under 50 °C. The result of the analysis of the hydrogenated dimer with mass spectrum analysis and nuclear magnetic resonance spectrum analysis showed bicyclo[2.2.1]heptane derivative represented by said formula (VII). Table 1 shows the measured result of general properties and the traction coefficient of the fraction.

### Example 7 (Reference Example)

200 g of hydrogenated dimer was yielded by replacing the catalyst of 8 g of diethyl ether complex of boron trifluoride for dimerization to 8 g of a 1.5 water complex of boron trifluoride by reaction under 10 °C. The result of the analysis of the hydrogenated dimer with mass spectrum analysis and nuclear magnetic resonance spectrum analysis showed bicyclo[2.2.1]heptane derivative represented by said formula (VII). Table 1 shows the measured result of general properties and the traction coefficient of the fraction.

### Comparative Example 1

240 g of hydrogenated dimer was yielded by replacing the hydrogenating reaction under the condition of 250 °C for 6 hours to the condition of 160 °C for 4 hours . The result of the analysis of the hydrogenated dimer with mass spectrum analysis and nuclear magnetic resonance spectrum analysis showed bicyclo[2.2.1]heptane derivative represented by said formula (VII). Table 1 shows the result of measurement of general properties and the traction coefficient of the fraction.

### Comparative Example 2

Crotonaldehyde (350.5 g = 5 mol) and dicyclopentadiene (198.3 g = 1 . 5 mol) were put in 1 L, stainless steel-made autoclave to react by mixing for 3 hours under 170 °C. The reaction solution was cooled to a room temperature, 2 g of a 5% ruthenium carbon catalyst [NE Chem Cat, K.K. made] was added, and finally, hydrogenation was carried out under a hydrogen pressure of 6.06 MPaG (70 kg/ cm²G) and reaction temperature of 180 °C for 4 hours. After cooling, the catalyst was filtered to separate, the filtrate was distilled under reduced pressure to yield 242 g of a fraction under 70 °C/119.9 Pa (0.9 mmHg). The result of mass spectrum analysis and nuclear magnetic resonance spectrum analysis of the fraction showed that the fraction is 2- hydroxy methyl-3-methylbicyclo[2.2.1]heptane and 3-hydroxy methyl-2-methylbicyclo[2.2.1]heptane.

Next, 15 g of γ-alumina [Nikka Seikou, K.K. made, Norton Alumina SA- 6273] was put in a quartz glass-made flow reaction tube under atmospheric pressure, with an outer diameter of 20 mm and a length of 500 mm to start dehydration reaction going under a reaction temperature of 270 °C and weight hourly space velocity (WHSV) = 1.07 hr⁻¹, and finally, 196 g of the dehydrating reaction product of 2- hydroxy methyl-3-methylbicyclo[2.2.1]heptane and 3-hydroxy methyl-2-methylbicyclo[2.2.1]heptane were yielded containing 65 weight percent of 2-methylene-3-methyl bicyclo[2.2.1]heptane and 3-methylene-2-methylbicyclo [2.2.1]heptane and 28 weight percent of 2,3-dimethyl bicyclo[2.2.1]hept-2-ene.

### Production of hydrogenated dimer

Activated clay (9.5 g) [Mizusawa Kagaku, K.K. made Galeon Earth NS] and the olefin compound (190 g) yielded from said steps were put in 500 ml four-neck flask and stirred under 145 °C for 3 hours to start dimerization reaction going. Activated clay was filtered from the reaction mixture, 6 g of nickel/diatomite catalyst [Nikki Kagaku, K.K. made, N-113) was added for hydrogenation in 1-L autoclave, and finally, hydrogenation reaction was started under the condition of 3.922 MPaG (40 kg/cm²G) of hydrogen pressure, reaction temperature of 160 °C, and reaction time of 4 hours. After the completion of reaction, the catalyst was removed by filtration to distill the filtrate under reduced pressure to yield 116 g of the hydrogenated dimer of 126 - 128 °C b.p. 26.6 Pa (0.2 mmHg) fraction. The result of the analysis of the hydrogenated dimer with mass spectrum analysis and nuclear magnetic resonance spectrum analysis showed bicyclo[2.2.1]heptane derivative represented by formula (VII) . The table 1 shows the measured result of general properties and the traction coefficient of the dimer hydride.

### Comparative Example 3

280 g of hydrogenated dimer was yielded by replacing the condition of dimerization reaction under 50 °C in the Example 6 to a condition under 100 °C. The result of the analysis of the hydrogenated dimer with mass spectrum analysis and nuclear magnetic resonance spectrum analysis showed bicyclo[2.2.1]heptane derivative represented by said formula (VII). Table 1 shows the result of measurement of general properties and the traction coefficient of the fraction.

The traction coefficient was measured by using a twin-cylinder friction tester in said Examples. The traction coefficient was known by measuring tangential force, that is, traction force, generated between these two cylinders with a constant speed for the one of contacting two cylinders with a same size (a driven, Japanese drum-shaped, cylinder with the radius of curvature of 10 mm and a driving cylinder with a flat type without crowning; diameter of 52 mm and thickness of 6 mm) and with a continuously changed rotation speed of the other, applying 98.0 N with a weight fitted to the contact point of both the cylinders. The cylinders were made by polishing to a mirror-smooth state of bearing steel SUJ-2 and showed average peripheral speed in girth of 6.8 m/s, and the maximum Herzian contact pressure of 1.23 GPa. The traction coefficient under fluid temperature (oil temp) 140 °C, was measured by heating an oil tank to increase the oil temperature from 40 °C to 140 °C and the traction coefficient was known at slide/oil ratio of 5%.

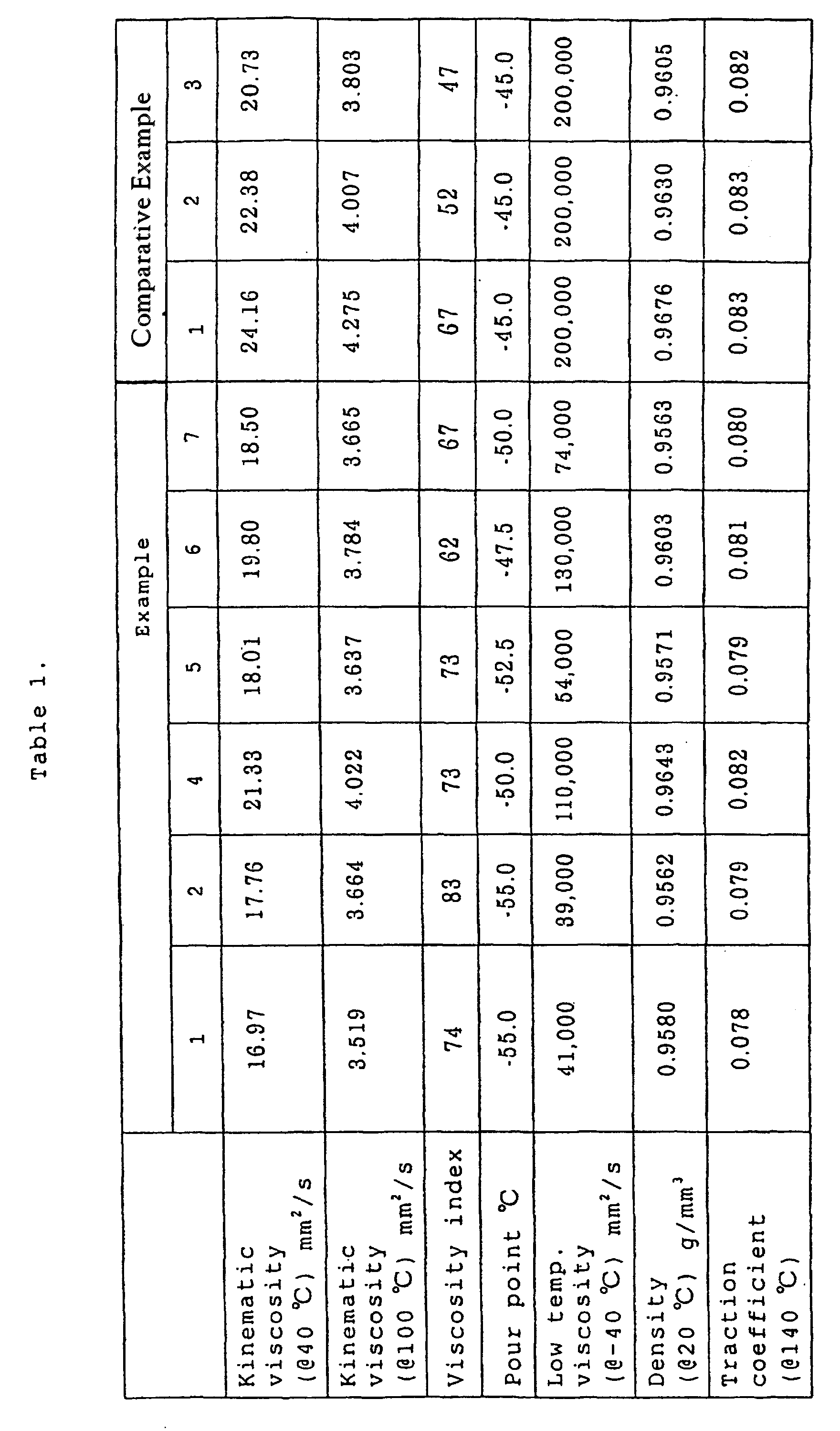

Bicyclo[2.2.1]heptane derivatives or fluid for traction drive according to the invention have a high traction coefficient under high temperature and an excellent characteristic of viscosity under low temperature to allow their use as a CVT oil for a traction drive in the world from cold and tropical regions. Various bicyclo[2.2.1]heptane derivatives can be efficiently prepared by the method according to the invention.

## Claims

1. Exo-2-methyl-exo-3-methyl-endo-2-[(endo-2-methylbicyclo [2.2.1]hept-exo-3-yl)methyl]bicyclo[2.2.1]heptane of formula (I), 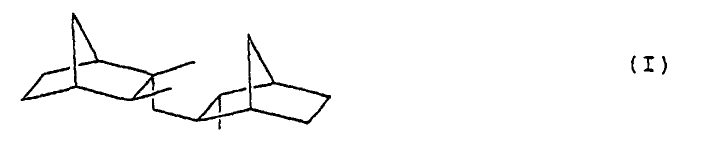 Exo-2-methyl-exo-3-methyl-endo-2[(endo-3-methylbicyclo[2.2.1]-hept-exo-2-yl)methyl] bicyclo[2.2.1]heptane of formula (II), 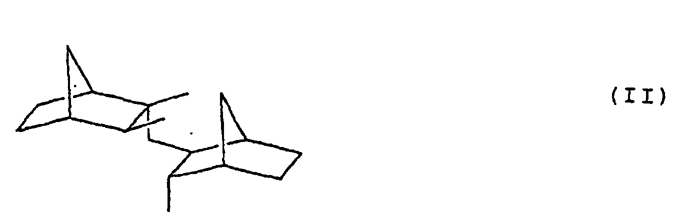 endo-2-methyl-exo-3-methyl-exo-2-[(exo-3-methylbicyclo-[2.2.1]hept-exo-2-yl)methyl]bicyclo[2.2.1]heptane of formula (III), 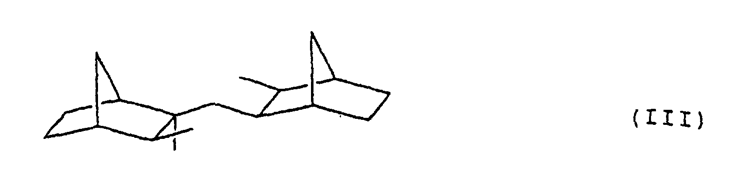 endo-2-methyl-exo-3-methyl-exo-2-[(exo-2-methylbicyclo[2.2.1]-hept-exo-3-yl)methyl]bicyclo[2.2.1]heptane of formula (IV), 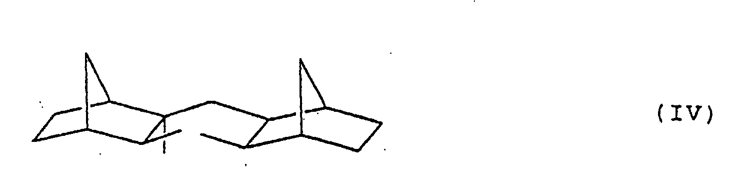 endo-2-methyl-exo-3-methyl-exo-2-[(endo-3-methylbicyclo-[2.2.1]hept-endo-2-yl)methyl]bicyclo[2.2.1]heptane of formula (V), 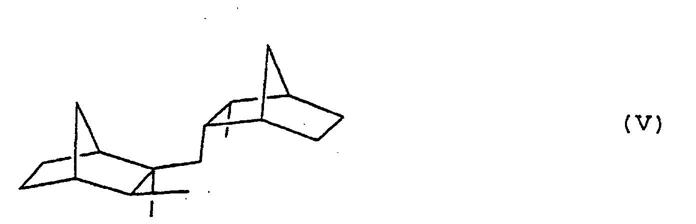 or endo-2-methyl-exo-3-methyl-exo-2-[(endo-2-methylbicyclo-[2.2.1]hept-endo-3-yl)methyl]bicyclo[2.2.1]heptane of formula (VI)

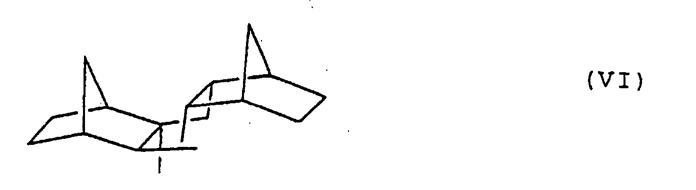

2. A mixture of exo-2-methyl-exo-3-methyl-endo-2[(endo-3-methylbicyclo[2.2.1]hept-exo-2-yl)methyl]-bicyclo-[2.2.1]heptane of formula II as defined in claim 1 and exo-2-methyl-exo-3-methyl-endo-2-[(endo-2-methyl-bicyclo[2.2.1]hept-exo-3-yl)methyl]bicyclo[2.2.1]heptane of formula I as defined in claim 1.

3. A mixture of endo-2-methyl-exo-3-methyl-exo-2-[(exo-3-methyl-bicyclo[2.2.1]hept-exo-2-yl)methyl]-bicyclo[2.2.1]heptane of formula III as defined in claim 1 and endo-2-methyl-exo-3-methyl-exo-2-[(exo-2-methylbicyclo[2.2.1]hept-exo-3-yl)-methyl]bicyclo-[2.2.1]heptane of formula IV as defined in claim 1.

4. A mixture of endo-2-methyl-exo-3-methyl-exo-2-[(endo-3-methyl-bicyclo[2.2.1]hept-endo-2-yl)methyl] bicyclo[2.2.1]heptane of formula V as defined in claim 1 and endo-2-methyl-exo-3-methyl-exo-2-[(endo-2-methylbicyclo[2.2.1]hept-endo-3-yl)methyl]bicyclo-[2.2.1] heptane of formula VI as defined in claim 1.

5. A method for preparing a bicyclo[2.2.1]heptane derivative represented by any of the formulae (I) to (VI) in claim 1,
**characterized by** dimerization of a methylene- and methyl-substituted bicyclo[2.2.1]heptane ring compound and/ or a methyl-substituted bicyclo[2.2.1]heptene ring compound in the presence of an acid catalyst at 60 °C or lower temperature and hydrogenating the produced dimer in the presence of a catalyst for hydrogenation at 200 - 300 °C

6. A method for preparing the bicyclo[2.2.1]heptane derivative according to claim 5, wherein the methylene- and methyl-substituted bicyclo[2.2.1]heptane ring compound is 2-methylene-3-methylbicyclo [2.2.1]heptane and/or 3-methylene-2-methylbicyclo[2.2.1]heptane.

7. A method for preparing the bicyclo[2.2.1]heptane derivative according to claim 5, wherein the methyl-substituted bicyclo[2.2.1]heptene ring compound is 2,3-dimethylbicyclo-[2.2.1] hept-2-ene.

8. A method for preparing the bicyclo[2.2.1]heptane derivative according to claim 5, wherein the acid catalyst is a Lewis acid.

9. A method for preparing the bicyclo[2.2.1]heptane derivative according to claim 5, wherein the catalyst for hydrogenation is a nickel catalyst.

10. A fluid for a traction drive, **characterized by** consisting of a synthetic oil having physical properties:
(1) Molecular weight: 210 or heavier.
(2) Kinematic viscosity at 40 °C : 10 - 25 mm²/s
(3) Viscosity index: 60 or higher
(4) Pour point: -40 °C or lower
(5) Density at 20 °C: 0.93 g/cm³ or higher
(6) Traction coefficient at 140 °C: 90% or higher of the coefficient of 2,4-dicyclohexyl-2-methylpentane
and wherein said synthetic oil is at least one selected from the group consisting of the compounds according to formula (I) to (VI) defined in claim 1.

11. A fluid for a traction drive consisting of at least one compound selected from the group of compounds according to claim 1.

## Patentansprüche

1. Exo-2-methyl-exo-3-methyl-endo-2-[(endo-2-methylbicyclo[2.2.1]hept-exo-3-yl)methyl]bicyclo[2.2.1]heptan der Formel (I), 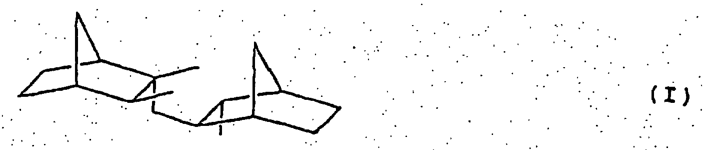 Exo-2-methyl-exo-3-methyl-endo-2-[(endo-3-methylbicyclo[2.2.1]hept-exo-2-yl)methyl]bicyclo[2.2.1]heptan der Formel (II), 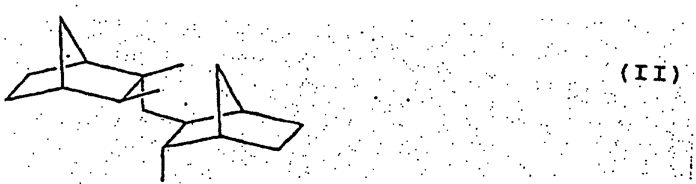 Endo-2-methyl-exo-3-methyl-exo-2-[(exo-3-methylbicyclo[2.2.1]hept-exo-2-yl)methyl]bicyclo[2.2.1]heptan der Formel (III), 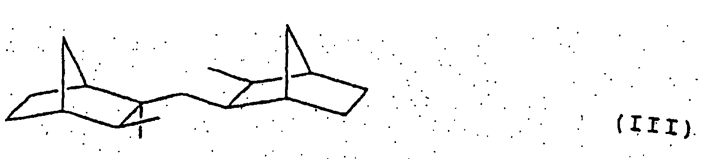 Endo-2-methyl-exo-3-methyl-exo-2-[(exo-2-methylbicyclo[2.2.1]hept-exo-3-yl)methyl]bicyclo[2.2.1]heptan der Formel (IV), 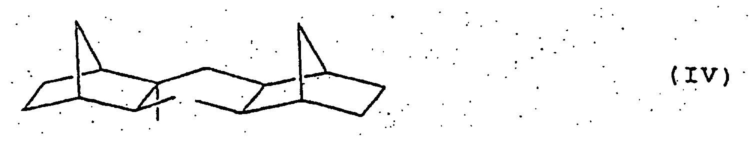 Endo-2-methyl-exo-3-methyl-exo-2-[(endo-3-methylbicyclo[2.2.1]hept-endo-2-yl)methyl]bicyclo[2.2.1]heptan der Formel (V), 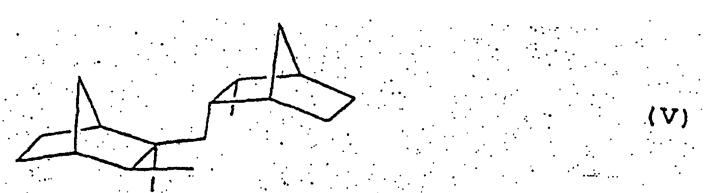 oder Endo-2-methyl-exo-3-methyl-exo-2-[(endo-2-methylbicyclo[2.2.1]hept-endo-3-yl)methyl]bicyclo[2.2.1]heptan der Formel (VI)

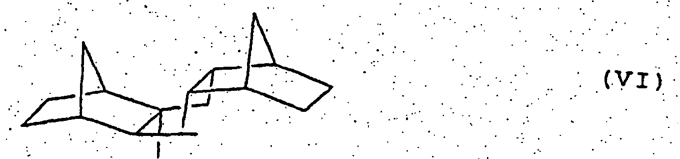

2. Eine Mischung von Exo-2-methyl-exo-3-methyl-endo-2-[(endo-3-methylbicyclo[2.2.1]hept-exo-2-yl)methyl]bicyclo[2.2.1]heptan der Formel II, wie in Anspruch 1 definiert, und Exo-2-methyl-exo-3-methyl-endo-2-[(endo-2-methylbicyclo[2.2.1]hept-exo-3-yl)methyl]bicyclo[2.2.1]heptan der Formel I, wie in Anspruch 1 definiert.

3. Eine Mischung von Endo-2-methyl-exo-3-methyl-exo-2-[(exo-3-methylbicyclo[2.2.1]hept-exo-2-yl)methyl]bicyclo[2.2.1]heptan der Formel III, wie in Anspruch 1 definiert, und Endo-2-methyl-exo-3-methyl-exo-2-[(exo-2-methylbicyclo[2.2.1]hept-exo-3-yl)methyl]bicyclo[2.2.1]heptan der Formel IV, wie in Anspruch 1 definiert.

4. Eine Mischung von Endo-2-methyl-exo-3-methyl-exo-2-[(endo-3-methylbicyclo[2.2.1]hept-endo-2-yl)methyl]bicyclo[2.2.1]heptan der Formel V, wie in Anspruch 1 definiert, und Endo-2-methyl-exo-3-methyl-exo-2-[(endo-2-methylbicyclo[2.2.1]hept-endo-3-yl)methyl]bicyclo[2.2.1]heptan der Formel VI, wie in Anspruch 1 definiert.

5. Verfahren zur Herstellung eines Bicyclo[2.2.1]heptan-Derivates, das durch eine der Formeln (I) bis (VI) im Anspruch 1 dargestellt ist, **dadurch gekennzeichnet, dass** eine Methylen- und Methyl-substituierte Bicyclo[2.2.1]heptan-Ringverbindung und/oder eine Methyl-substituierte Bicyclo[2.2.1]hepten-Ringverbindung in Gegenwart eines Säurekatalysators bei 60° C oder einer niedrigeren Temperatur dimerisiert und das hergestellte Dimer in Gegenwart eines Katalysators für die Hydrierung bei 200 - 300° C hydriert wird.

6. Verfahren zur Herstellung des Bicyclo[2.2.1]heptan-Derivates nach Anspruch 5, wobei die Methylen- und Methyl-substituierte Bicyclo[2.2.1]heptan-Ringverbindung 2-Methylen-3-methylbicyclo[2.2.1]heptan und/oder 3-Methylen-2-methylbicyclo[2.2.1]heptan ist.

7. Verfahren zur Herstellung des Bicyclo[2.2.1]heptan-Derivates nach Anspruch 5, wobei die Methyl-substituierte Bicyclo[2.2.1]hepten-Ringverbindung 2,3-Dimethylbicyclo- [2.2.1]hept-2-en ist.

8. Verfahren zur Herstellung des Bicyclo[2.2.1]heptan-Derivates nach Anspruch 5, wobei der Säurekatalysator eine Lewis-Säure ist.

9. Verfahren zur Herstellung des Bicyclo[2.2.1]heptan-Derivates nach Anspruch 5, wobei der Katalysator für die Hydrierung ein Nickelkatalysator ist.

10. Flüssigkeit für einen Traktionsantrieb, **dadurch gekennzeichnet, dass** sie aus einem synthetischen Öl mit den folgenden physikalischen Eigenschaften besteht:
(1) relative Molekülmasse: 210 oder schwerer,
(2) kinematische Viskosität bei 40° C: 10 - 25 mm²/Sek.,
(3) Viskositätsindex: 60 oder höher,
(4) Fließpunkt: -40° C oder niedriger,
(5) Dichte bei 20° C: 0,93 g/cm³ oder größer,
(6) Traktionskoeffizient bei 140° C: 90 % oder mehr des Koeffizienten von 2,4-Dicyclohexyl-2-methylpentan,
und wobei das synthetische Öl wenigstens ein Öl ist, welches aus der Gruppe ausgewählt ist, die aus den Verbindungen gemäß den Formeln (I) bis (VI) besteht, welche im Anspruch 1 definiert sind.

11. Flüssigkeit für einen Traktionsantrieb, die wenigstens aus einer Verbindung besteht, die aus der Gruppe der Verbindungen gemäß Anspruch 1 ausgewählt ist.

## Revendications

1. Exo-2-méthyl-exo-3-méthyl-endo-2-[(endo-2-méthylbicyclo [2.2.1] hept-exo-3-yl) méthyl] bicyclo [2.2.1] heptane de formule (I). 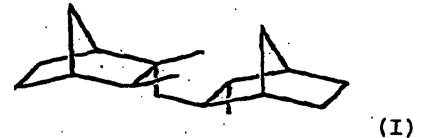 exo-2-méthyl-exo-3-méthyl-endo-2-[(endo-3-méthylbicyclo [2.2.1] hept-exo-2-yl) méthyl] bicyclo [2.2.1] heptane de formule (II), 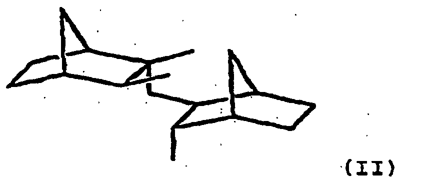 endo-2-méthyl-exo-3-méthyl-exo-2-[ (exo-3-méthylbicyclo [2.2.1] hept-exo-2-yl) méthyl] bicyclo [2.2.1] heptane de formule (III), 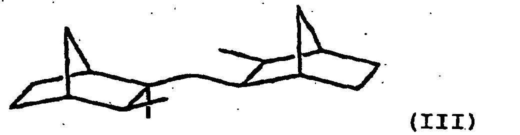 endo-2-méthyl-exo-3-méthyl-exo-2-[(exo-2-méthylbicyclo [2.2.1] hept-exo-3-yl) méthyl] bicyclo [2.2.1] heptane de formule (IV), 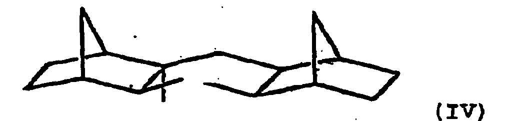 endo-2-méthyl-exo-3-méthyl-exo-2-[(endo-3-méthylbicyclo [2.2.1] hept-endo-2-yl) méthyl] bicyclo [2.2.1] heptane de formule (V), 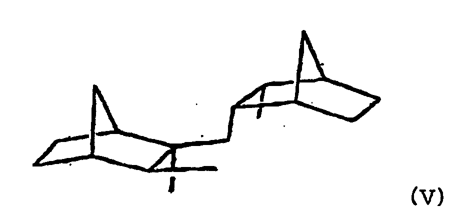 ou endo-2-méthyl-exo-3-méthyl-exo-2-[(endo-2-méthylbicyclo [2.2.1] hept-endo-3-yl) méthyl] bicyclo [2.2.1] heptane de formule (VI),

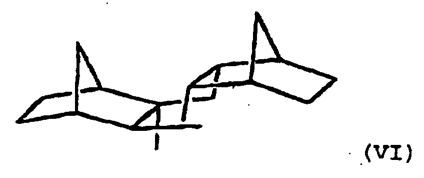

2. Mélange d'exo-2-méthyl-exo-3-méthyl-endo-2-[(endo-3-méthylbicyclo [2.2.1] hept-exo-2-yl) méthyl] bicyclo [2.2.1] heptane de formule II tel que défini selon la revendication 1, et d'exo-2-méthyl-exo-3-méthyl-endo-2-[(endo-2-méthylbicyclo [2.2.1] hept-exo-3-yl) méthyl] bicyclo [2.2.1] heptane de formule I tel que défini selon la revendication 1.

3. Mélange d'endo-2-méthyl-exo-3-méthyl-exo-2-[(exo-3-méthylbicyclo [2.2.1] hept-exo-2-yl) méthyl] bicyclo [2.2.1] heptane de formule III tel que défini selon la revendication 1, et d'endo-2-méthyl-exo-3-méthyl-exo-2-[(exo-2-méthylbicyclo [2.2.1] hept-exo-3-yl) méthyl] bicyclo [2.2.1] heptane de formule IV tel que défini selon la revendication 1.

4. Mélange d'endo-2-méthyl-exo-3-méthyl-exo-2-[(endo-3-méthylbicyclo [2.2.1] hept-endo-2-yl) méthyl] bicyclo [2.2.1] heptane de formule V tel que défini selon la revendication 1, et d'endo-2-méthyl-exo-3-méthyl-exo-2-[(endo-2 -méthylbicyclo [2.2.1] hept-endo-3-yl) méthyl] bicyclo [2.2.1] heptane de formule VI tel que défini selon la revendication 1.

5. Procédé de préparation d'un dérivé de bicyclo [2.2.1] heptane représente par l'une quelconque des formules (I) à (VI) selon la revendication 1, **caractérisé par** la dimérisation d'un composé cyclique bicyclo [2.2.1] heptane substitué par un méthylène et un méthyle et/ou un composé cyclique bicyclo [2.2.1] heptène substitué par un méthyle, en présence d'un catalyseur acide, à 60°C ou une température inférieure, et l'hydrogénation du dimère produit en présence d'un catalyseur pour l'hydrogénation à 200 à 300°C.

6. Procédé pour la préparation du dérivé de bicyclo [2.2.1] heptane selon la revendication 5, dans lequel le composé cyclique bicyclo [2.2.1] heptane substitué par un méthylène et un méthyle est le 2-méthylène-3-méthylbicyclo [2.2.1] heptane et/ou le 3-méthylène-2-méthylbicyclo [2.2.1] heptane.

7. Procédé pour la préparation d'un dérivé de bicyclo [2.2.1] heptane selon la revendication 5, dans lequel le composé cyclique bicyclo [2.2.1] heptène substitué par un méthyle, est le 2,3-diméthylbicyclo [2.2.1] hept-2-ène.

8. Procédé pour la préparation du dérivé de bicyclo [2.2.1] heptane selon la revendication 5, dans lequel le catalyseur acide est un acide de Lewis.

9. Procédé pour la préparation d'un dérivé de bicyclo [2.2.1] heptane selon la revendication 5, dans lequel le catalyseur pour l'hydrogénation est un catalyseur au nickel.

10. Fluide pour une commande de traction, **caractérisé en ce qu'**il est constitué d'une huile synthétique ayant les propriétés physiques de :
(1) poids moléculaire : 210 ou supérieur'
(2) viscosité cinématique à 40°C : 10 à 25 mm²/s
(3) indice de viscosité : 60 ou supérieure
(4) point de figeage : -40°C ou inférieur
(5) densité à 20°C : 0,93 g/cm³ ou supérieure
(6) coefficient de traction à 140°C : 90 % ou supérieur au coefficient du 2,4-dicyclohexyl-2-méthylpentane
et dans lequel ladite huile synthétique est au moins choisie dans le groupe constitué par les composés en accord avec les formules (I) à (VI) définies selon la revendication 1.

11. Fluide pour une commande de traction constitué d'au moins un composé choisi dans le groupe des composés selon la revendication 1.
